# EUROPEAN PATENT APPLICATION

(11) **EP 4 789 551 A1**
(43) Date of publication of application: **12.08.2026**
(21) Application number: 25880935.9
(22) Date of filing: 19.12.2025
(51) Int. Cl.: A23L 33/105, A23L 29/00, A23L 19/10, A23P 10/00

(54) **COMPOSITION FOR ENHANCING IMMUNITY COMPRISING ENZYME-TREATED PRODUCT OF POLYSACCHARIDES OR PURIFIED FRACTION THEREOF**

(30) Priority: 20.12.2024 KR 20240193354
(71) Applicant: Kolmar BNH Co., Ltd, Sejong 30003 (KR)
(72) Inventor: SHIN, Ji Eun, Seocho-gu, Seoul 06800 (KR); PARK, Su Bin, Seocho-gu, Seoul 06800 (KR); KIM, Ju Gyeong, Seocho-gu, Seoul 06800 (KR); KIM, Sang Back, Seocho-gu, Seoul 06800 (KR); CHOI, En Jin, Seocho-gu, Seoul 06800 (KR); KIM, Young Mu, Seocho-gu, Seoul 06800 (KR)
(74) Representative: Regimbeau
(86) International application number: PCT/KR2025/022395
(87) International publication number: WO 2026/135371

(57) **Abstract**

The present disclosure relates to a composition for enhancing immunity, comprising, as an active ingredient, an enzyme-treated polysaccharide derived from a mixed extract of angelica, cnidium, and paeonia; or a novel purified fraction thereof.

The composition for enhancing immunity according to the present disclosure, comprising, as an active ingredient, an enzyme-treated polysaccharide of a mixed extract of angelica, cnidium, and paeonia; or a novel purified fraction thereof, is composed of sugars of specific types and contents, and treating macrophages, splenocytes, and NK cells therewith significantly increases the activities of these cells. As such, the same can be applied as an immunoenhancer or a food composition for enhancing immunity.

## Description

### [Technical Field]

The present disclosure relates to a composition for enhancing immunity, comprising, as an active ingredient, an enzyme-treated polysaccharide derived from a mixed extract of angelica, cnidium, and paeonia; or a purified fraction thereof.

### [Background Art]

The immune response is a self-defense mechanism for protecting the body, and comprises processes of removing or neutralizing various antigens introduced from outside the body as well as unnecessary metabolic by-products within the body, thereby maintaining homeostasis and suppressing occurrence of disease. Immune responses in the body are broadly classified into innate immunity, which is present from birth, and acquired immunity, which is obtained through adaptation to life, *etc*.

Innate immunity, also referred to as natural immunity, responds to antigens in a non-specific manner and does not exhibit a specific memory function. Such innate immunity in fact defends against the majority of infections. Components of the innate immune system include the skin and mucosal tissues, strongly acidic gastric acid, complements present in the blood, *etc*. that prevent antigen invasion, as well as cells such as macrophages and polymorphonuclear leukocytes responsible for phagocytosis, and NK cells.

Acquired immunity, also called adaptive immunity, can remember an antigen upon initial exposure and respond in a specific manner upon re-exposure, thereby effectively eliminating the antigen. Such acquired immunity is divided into humoral immunity and cell-mediated immunity. Humoral immunity involves B lymphocytes that, upon recognition of an antigen, undergo differentiation and secrete antibodies, which mainly eliminate infectious bacteria. Cell-mediated immunity involves thymus-derived T lymphocytes (T cells) that recognize antigens and either secrete lymphokines or directly kill infected cells, and such cell-mediated immunity primarily functions to eliminate cells infected with viruses or bacteria capable of growing intracellularly.

Among the cells involved in immune responses, macrophages regulate immune phenomena through the secretion of various cytokines during the processes of phagocytosing and eliminating bacteria or foreign substances. As cells playing a central role in immune responses to antigens, macrophages are associated with antigen presentation and non-specific immune actions of lymphocytes, and also exhibit direct cytotoxic activity against tumor cells. In addition, substances that react to Toll-like receptors (TLRs) (*e.g.,* LPS or natural products) are known to activate macrophages to produce cytokines such as IL-1, IL-6, IL-10, IL-12, and TNF-α, which can regulate secondary immune responses such as proliferation of T cells and B cells, phagocytic activity, and defense against microbial infection. IL-1, IL-6, and TNF-α are representative cytokines induced by macrophages, play central roles in inflammatory responses to bacterial infection, and are known to increase in amount at inflammatory lesions. IL-6 has been reported to act cooperatively with IL-1 in the differentiation of T cells and B cells, and TNF-α is known to have antiviral activity and to play important roles in various biological responses. In addition, IL-12 is known as a cytokine that activates NK cells and induces Th1-type immune responses, thereby enhancing responsiveness to xenobiotics.

Natural killer cells (NK cells) play important roles in innate immune responses that directly attack cells infected by a host or tumor cells, and in adaptive immune responses that activate cytotoxic T cells or B cells through cytokine secretion (Advances in Immunology, 2014, 137-170). NK cells are activated by IFNs or cytokines derived from macrophages, and activated NK cells secrete cytokines such as IFN-γ and TNF-α, thereby promoting apoptosis of tumor cells or contributing to the activation of other immune cells. The activity of NK cells is regulated by a balance of signal transduction in the presence of various activating and inhibitory receptors, and through such regulation, NK cells do not attack normal cells and selectively eliminate infected cells (Cancer Immunol. Immunother. 2014; 63(6): 571-580).

Regulation of NK cell activity is known to be associated with various diseases such as cancer or immune disorders, and there is an emerging need for effective methods for enhancing immunity using NK cells.

Meanwhile, natural products are diverse mixtures of low molecular weight substances such as alkaloids, flavonoids, terpenoids, and saponins, as well as high molecular weight substances such as polysaccharides, proteins, and tannins, and exhibit low toxicity. Research in various pharmaceutical products or functional foods utilizing polysaccharides derived therefrom is increasingly being conducted; however, further studies are still required with respect to the pharmacological activities and immunoenhancing activities of high molecular weight carbohydrates.

In such context, the present inventors have confirmed that treating macrophages, splenocytes, and NK cells with an enzyme-treated polysaccharide derived from a mixed extract of angelica, cnidium, and paeonia; or a purified fraction thereof composed of sugars of specific types and contents results in a significant increase in the activities of these cells. In addition, the inventors have newly elucidated that the same can be utilized as an immunoenhancer or an anti-cancer immunomodulator, thereby completing the present invention.

### [Disclosure]

### [Technical Problem]

An object of the present disclosure is to provide a composition for enhancing immunity or modulating anti-cancer immunity, comprising, as an active ingredient, an enzyme-treated polysaccharide of a mixed extract of angelica, cnidium, and paeonia; or a purified fraction thereof.

Another object of the present disclosure is to provide a food composition for enhancing immunity or modulating anti-cancer immunity, comprising, as an active ingredient, an enzyme-treated polysaccharide of a mixed extract of angelica, cnidium, and paeonia; or a purified fraction thereof.

Still another object of the present disclosure is to provide a method for activating cells *in vitro*, *ex vivo*, or both, comprising treating macrophages, splenocytes, or NK cells with an enzyme-treated polysaccharide of a mixed extract of angelica, cnidium, and paeonia; or a purified fraction thereof.

Still another object of the present disclosure is to provide a use of a composition comprising, as an active ingredient, an enzyme-treated polysaccharide of a mixed extract of angelica, cnidium, and paeonia; or a purified fraction thereof for enhancing immunity or modulating anti-cancer immunity.

Still another object of the present disclosure is to provide a method for enhancing immunity or modulating anti-cancer immunity, comprising administering to a subject a composition comprising, as an active ingredient, an enzyme-treated polysaccharide of a mixed extract of angelica, cnidium, and paeonia; or a purified fraction thereof.

### [Technical Solution]

To solve the above problem, an aspect of the present disclosure is a composition for enhancing immunity or modulating anti-cancer immunity, comprising, as an active ingredient, an enzyme-treated polysaccharide of a mixed extract of angelica, cnidium, and paeonia; or a purified fraction thereof.

In one specific embodiment, the immunity is characterized by being innate immunity, acquired immunity, or a combination thereof.

In another specific embodiment, the mixed extract is characterized by being extracted with a solvent selected from the group consisting of water, a linear or branched alcohol having 1 to 6 carbon atoms, an organic solvent, and a mixed solvent thereof.

In a specific embodiment according to any one of the preceding specific embodiments, the mixed extract is characterized by comprising angelica, cnidium, and paeonia at a weight ratio of 1:1.2 to 3:1.2 to 3.

In a specific embodiment according to any one of the preceding specific embodiments, the mixed extract is characterized by being a mixed extract of *Angelica sinensis* (Oliv.) Diels, *Ligusticum chuanxiong* Hort., and *Paeonia lactiflora* Pallas.

In a specific embodiment according to any one of the preceding specific embodiments, the polysaccharide is characterized by being an ethanol polysaccharide.

In a specific embodiment according to any one of the preceding specific embodiments, the enzyme is characterized by comprising pectinase.

In a specific embodiment according to any one of the preceding specific embodiments, the enzyme-treated polysaccharide or purified fraction thereof is characterized by comprising a neutral sugar, a uronic acid, a protein, a ketodeoxy-octonate (KDO)-like material, and a polyphenol.

In a specific embodiment according to any one of the preceding specific embodiments, the enzyme-treated polysaccharide is characterized by comprising 68.1% to 73.5% neutral sugar, 11.4% to 14.8% uronic acid, 5.6% to 6.8% protein, 2.1% to 2.9% KDO-like material, and 6.4% to 8.4% polyphenol.

In a specific embodiment according to any one of the preceding specific embodiments, the purified fraction is characterized by comprising 66.1% to 80.7% neutral sugar, 16.2% to 19% uronic acid, 1.7% to 1.9% protein, 0.8% to 2.6% KDO-like material, and 4.9% to 6.1% polyphenol.

In a specific embodiment according to any one of the preceding specific embodiments, the enzyme-treated polysaccharide or purified fraction thereof is characterized by comprising at least one selected from the group comprising rhamnose, fucose, arabinose, mannose, galactose, glucose, glucuronic acid, and galacturonic acid.

In a specific embodiment according to any one of the preceding specific embodiments, the enzyme-treated polysaccharide is characterized by comprising 2.7 mol% to 3.7 mol% rhamnose, 8.1 mol% to 8.7 mol% arabinose, 2.3 mol% to 2.7 mol% mannose, 35.8 mol% to 36.8 mol% galactose, 39.5 mol% to 39.9 mol% glucose, and 9.5 mol% to 10.5 mol% galacturonic acid.

In a specific embodiment according to any one of the preceding specific embodiments, the purified fraction is characterized by comprising 3.5 mol% to 4.5 mol% rhamnose, 13.9 mol% to 14.5 mol% arabinose, 1.5 mol% to 1.7 mol% mannose, 69.9 mol% to 71.7 mol% galactose, 1.1 mol% to 2.1 mol% glucose, 1.4 mol% to 1.6 mol% glucuronic acid, and 5 mol% to 7.4 mol% galacturonic acid.

In a specific embodiment according to any one of the preceding specific embodiments, the enzyme-treated polysaccharide or purified fraction thereof is characterized by activating macrophages, splenocytes, or natural killer cells.

In a specific embodiment according to any one of the preceding specific embodiments, the enzyme-treated polysaccharide or purified fraction thereof is characterized by increasing immune-related cytokines.

In a specific embodiment according to any one of the preceding specific embodiments, the cytokine is characterized by being at least one selected from the group consisting of IL-2, IFN-γ, IL-4, IL-10, TNF-α, IL-12, and IL-6.

In a specific embodiment according to any one of the preceding specific embodiments, the composition is characterized by further comprising a mixed extract of angelica, cnidium, and paeonia.

In a specific embodiment according to any one of the preceding specific embodiments, the mixed extract and the enzyme-treated polysaccharide or purified fraction thereof are characterized by being mixed at a weight ratio of 60 to 70:30 to 40.

In a specific embodiment according to any one of the preceding specific embodiments, the enzyme-treated polysaccharide or purified fraction thereof is characterized by being comprised in an amount of 0.001 wt% to 99 wt% based on the total weight of the composition.

In a specific embodiment according to any one of the preceding specific embodiments, the composition is characterized by being a food composition.

In a specific embodiment according to any one of the preceding specific embodiments, the food composition is characterized by being prepared as a powder, granule, tablet, capsule, syrup, or beverage.

Another aspect of the present disclosure is a health supplement comprising a composition for enhancing immunity or modulating anti-cancer immunity, comprising an enzyme-treated polysaccharide of a mixed extract of angelica, cnidium, and paeonia; or a purified fraction thereof.

Still another aspect of the present disclosure is a method for activating cells *in vitro*, *ex vivo*, or both, comprising treating cells with an enzyme-treated polysaccharide of a mixed extract of angelica, cnidium, and paeonia; or a purified fraction thereof.

In one specific embodiment, the cells are characterized by being macrophages, splenocytes, NK cells, or a combination thereof.

Still another aspect of the present disclosure is a use of a composition comprising, as an active ingredient, an enzyme-treated polysaccharide of a mixed extract of angelica, cnidium, and paeonia; or a purified fraction thereof for enhancing immunity or modulating anti-cancer immunity.

Still another aspect of the present disclosure is a method for enhancing immunity or modulating anti-cancer immunity, comprising administering to a subject a composition comprising, as an active ingredient, an enzyme-treated polysaccharide of a mixed extract of angelica, cnidium, and paeonia; or a purified fraction thereof.

### [Advantageous Effects]

The composition for enhancing immunity according to the present disclosure, comprising, as an active ingredient, an enzyme-treated polysaccharide of a mixed extract of angelica, cnidium, and paeonia; or a novel purified fraction thereof, is composed of sugars of specific types and contents, and treating macrophages, splenocytes, and NK cells therewith significantly increases the activities of these cells. As such, it can be applied as an immunoenhancer or a food composition for enhancing immunity.

### [Brief Description of Drawings]

FIG. 1 shows results confirming the purity of an ethanol polysaccharide and an enzyme-treated product thereof according to one example of the present disclosure, as determined according to size-exclusion chromatography.
FIG. 2 shows results confirming the degree of purification of a purified fraction according to one example of the present disclosure, using an open column.
FIG. 3 shows results confirming the macrophage-activating effect of an enzyme-treated polysaccharide or a purified fraction thereof according to one example of the present disclosure.
FIG. 4 shows results confirming the macrophage-activating effect of four enzyme-treated polysaccharides according to one example of the present disclosure.
FIG. 5 shows results confirming the splenocyte-activating effect of an enzyme-treated polysaccharide or a purified fraction thereof according to one example of the present disclosure.
FIG. 6 shows results confirming the tumor cell-killing effect of natural killer cells induced by the purified fraction GHHE-I according to one example of the present disclosure. In particular, the E/T ratio refers to the ratio of NK cells isolated from the spleen (effector cells, E) to Yac-1 tumor cells (target cells, T) known to be sensitive to NK cells.

### [Modes for Carrying Out the Invention]

Each description and embodiment disclosed herein may also be applied to other descriptions and embodiments. That is, all combinations of various elements described herein fall within the scope of the present disclosure. Further, the scope of the present disclosure is not limited by the specific description described below.

Furthermore, those skilled in the art will recognize or be able to ascertain, using no more than routine experimentation, many equivalents to the specific aspects of the present disclosure described herein. Additionally, such equivalents are intended to be included in the present disclosure.

Moreover, throughout the specification of the present disclosure, when a part is said to "comprise" a certain component, this means that the part may further include other components, rather than excluding all other components, unless explicitly stated otherwise.

Hereinafter, the present disclosure is described in more detail.

The present disclosure is based on the novel observation that treating macrophages, splenocytes, and NK cells with an enzyme-treated polysaccharide derived from a mixed extract of angelica, cnidium, and paeonia; or a purified fraction thereof composed of sugars of specific types and contents results in a significant increase in the activities of these cells, and on the discovery that it can be utilized as an immunoenhancer or an anti-cancer immunomodulator.

An aspect of the present disclosure that is provided to achieve the above objects is a composition for enhancing immunity or modulating anti-cancer immunity, comprising, as an active ingredient, an enzyme-treated polysaccharide of a mixed extract of angelica, cnidium, and paeonia; or a purified fraction thereof.

As used herein, the term "angelica" refers to an angiosperm belonging to the *Apiaceae* family, which has long been used medicinally in East Asia, with its root portion most commonly utilized. Accordingly, the dried root of angelica is also interchangeably referred to as "angelica".

Depending on the origin, *Angelica sinensis* (Oliv.) Diels originating from China is distinguished from *Angelica gigas* Nakai in Korea and *Angelica acutiloba* (Siebold. & Zucc.) Kitag. or *Angelica acutiloba* (Siebold. & Zucc.) Kitag. var. sugiyamae Hikino root in Japan.

*Angelica sinensis* is warm in energy and has a sweet and spicy taste. In general, *Angelica sinensis* is sweeter and less spicy than *Angelica gigas*. The efficacy of *Angelica sinensis* is to replenish blood when there is a lack of blood, and angelica made of the root of *Angelica sinensis* has an excellent blood-replenishing effect. However, angelica made of the root of *Angelica gigas* is more effective in promoting blood circulation than in replenishing blood, and has strong anti-cancer and blood pressure-lowering effects. Pharmacologically, it is known that angelica promotes blood flow in coronary arteries and stimulates red blood cell production.

As used herein, the term "cnidium" refers to *Ligusticum chuanxiong* Hort. or *Cnidium officinale*, preferably refers to *Ligusticum chuanxiong* Hort., and is a perennial herb of the *Apiaceae* family, *Apiaceae* order, and *Dicotyledonous* group of dicotyledons commonly cultivated as a medicinal plant. Cnidium is effective for sedation, pain relief, and strengthening, and is used to treat headaches, anemia, and gynecological diseases. The rhizome of cnidium is dug up in September to November, dried in the sun with the leaves and stems removed, and then boiled and taken or made into pills or powders for use.

As used herein, the term "paeonia (*Paeonia lactiflora* Pallas)" refers to a perennial herb of the *Paeonia* genus in the *Paeoniaceae* family, a dicotyledonous plant growing in mountainous areas, and is used for horticultural purposes because of its beautiful flowers. Additionally, the root is used as a medicinal herb for pain, abdominal pain, menstrual cramps, amenorrhea, vomiting blood, anemia, and bruises. Paeonia has been cultivated as an ornamental plant in China since the Qin and Ming Dynasties, and its cultivation history is longer than that of *Paeonia suffruticosa* Andr. Dozens of varieties of Paeonia were recorded during the Song and Qing Dynasties, and are distributed in Korea, Mongolia, and East Siberia.

In the present disclosure, paeonia may include at least one selected from the group consisting of *Paeonia lactiflora* Pallas, *Paeonia obovata var. japonica*, *Paeonia* sect. *Paeonia*, *Paeonia lactiflora for. pilosella Nakai*, and *Paeonia lactiflora var. trichocarpa (Bunge) Stern*, and may be used without limitation regardless of the processing method.

The angelica, cnidium, and paeonia may be purchased commercially, collected from nature, or cultivated and used without limitation.

As used herein, the term "extract" refers to a mixed extract containing angelica, cnidium, and paeonia, and the mixed extract of angelica, cnidium, and paeonia may be obtained from various organs of natural, hybrid, and mutant plants. Specifically, the extract may be extracted from roots, aerial parts, stems, leaves, flowers, fruit bodies, and fruit peels as well as plant tissue cultures.

The "mixed extract" is a mixed extract comprising all of angelica, cnidium, and paeonia, and may be an extract obtained by first mixing the three substances and then subjecting the mixture to extraction, or may be an extract obtained by subjecting each substance to extraction individually and then mixing the extracts thus prepared. The term also includes, without limitation, any mixed extract prepared by any other known method capable of preparing a mixed extract.

The extract may be extracted from a pulverized product of each component with a solvent selected from the group consisting of water, a linear or branched alcohol having 1 to 6 carbon atoms, an organic solvent, and a mixed solvent thereof, but is not limited thereto. In addition, the extract may be an extract obtained by performing extraction in an extraction temperature range of 20°C to 100°C for an extraction period of about 1 hour to 10 days by an extraction method such as hot water extraction, cold extraction, reflux cooling extraction, or ultrasonic extraction. However, the extraction is not limited to the extraction temperature, extraction period, or extraction method above.

In a case where the solvent of the extract is water, the extract may include a cold water extract or a hot water extract.

The organic solvent is not particularly limited, and may be polyhydric alcohol such as glycerol, ethylene glycol, propylene glycol, and 1,3-butylene glycol; hydrocarbon-based solvents such as methyl acetate, ethyl acetate, benzene, n-hexane, diethyl ether, dichloromethane, and chloroform; and nonpolar organic solvents such as petroleum ether, methyl acetate, benzene, hexane, chloroform, methylene chloride, dimethyl ether, and ethyl acetate.

The solvent may also include an aqueous solution of an organic solvent. The concentration thereof is not particularly limited, but may be 1% to 99% (v/v), specifically 60% to 98% (v/v), more specifically 80% to 95% (v/v) or 85% to 95% (v/v), and still more specifically 90% to 95% (v/v).

The extract may include all of an extract; a diluted or concentrated extract; a dried product obtained by drying an extract; or modified or purified products thereof.

The extract may comprise angelica, cnidium, and paeonia at a weight ratio of 1 to 10:1 to 10:1 to 10, specifically a weight ratio of 1 to 5:1 to 5:1 to 5, more specifically a weight ratio of 1:1.2 to 3:1.2 to 3, still more specifically a weight ratio of 1:1.3 to 2:1.2 to 2, still more specifically a weight ratio of 1:1.4 to 1.7:1.2 to 1.6, still more specifically a weight ratio of 1:1.5 to 1.7:1.2 to 1.5, and still more specifically a weight ratio of 1:1.6:1.3.

In one specific embodiment, the extract may be a mixed extract comprising *Angelica sinensis* (Oliv.) Diels, *Ligusticum chuanxiong* Hort., and *Paeonia lactiflora* Pallas at a weight ratio of 1:1.6:1.3.

In the extract of the present disclosure, an extract of a known natural product or a known component that enhances or strengthens immunity may optionally be added.

As used herein, the term "polysaccharide" refers to a polysaccharide precipitated by adding a solvent to the mixed extract of angelica, cnidium, and paeonia. In particular, the polysaccharide is also called a polysaccharide and may comprise at least one polysaccharide.

In the present disclosure, the polysaccharide refers to the polysaccharide itself obtained by treating an extract with a solvent and precipitating without further purification, and may be used interchangeably with crude polysaccharide or GHHW-P.

In the present disclosure, the method for obtaining the polysaccharide is not particularly limited, and may be performed according to a method commonly used in the art. As a non-limiting example of the method, the polysaccharide may be obtained from the extract of angelica, cnidium, and paeonia of the present disclosure by treating the extract with a predetermined solvent, and specifically, the polysaccharide may be produced by adding an organic solvent to the extract of angelica, cnidium, and paeonia of the present disclosure, but the method is not limited thereto.

In the present disclosure, the type of solvent used to obtain the polysaccharide is not particularly limited, and any solvent known in the art may be used. Non-limiting examples of the solvent include water, a linear or branched alcohol having 1 to 6 carbon atoms, an organic solvent, or a mixed solvent thereof. These may be used alone or in a mixture of at least two, but the solvent is not limited thereto. Further, the organic solvent may be at least one selected from the group consisting of dichloromethane, diethyl ether, chloroform, and ethyl acetate, but is not limited thereto.

Specifically, the polysaccharide may be an ethanol polysaccharide, and more specifically a 90% to 95% ethanol polysaccharide.

As used herein, the term "enzyme-treated polysaccharide" refers to a product obtained by treating a polysaccharide, which has been obtained by treating an extract with a solvent and precipitating it, with an enzyme. In particular, the enzyme may comprise pectinase.

In one example, the enzyme-treated polysaccharide of the present disclosure comprises an enzyme-treated product obtained by treating a polysaccharide, precipitated by treating a mixed hot-water extract of angelica, cnidium, and paeonia with ethanol, with pectinase. The enzyme-treated polysaccharide of the present disclosure may be characterized in that it does not comprise other enzyme-treated products, as even when the same polysaccharide is used, enzyme-treated products obtained by treatment with enzymes other than pectinase, such as cellulase, protease, or amylase, exhibit only insignificant immunoenhancing activity.

The enzyme-treated polysaccharide is characterized in that its immunoenhancing activity is increased by treating an ethanol polysaccharide with an enzyme to remove an inactive fraction, namely the HG region.

The term "purified fraction" refers to a fraction obtained by purifying an enzyme-treated polysaccharide, obtained by treating a polysaccharide, which has been obtained by treating an extract with a solvent and precipitating it, with an enzyme. The purified fraction refers to a fraction purified according to the molecular weight of component sugars comprised therein, and may be used interchangeably with terms such as polysaccharide fraction, enzyme-treated product fraction, enzyme-treated polysaccharide fraction, purified polysaccharide fraction, or refined polysaccharide fraction. Specifically, the purified fraction may include GHHE-I, GHHE-II, and GHHE-III, and more specifically GHHE-I.

The purified fractions GHHE-I, GHHE-II, and GHHE-III refer to fractions obtained by purifying an enzyme-treated polysaccharide, obtained by treating a polysaccharide, which has been obtained by treating an extract with a solvent and precipitating it, with an enzyme; and may be fractionated according to molecular weight.

Specifically, the purified fraction GHHE-I is a fraction having a higher molecular weight than GHHE-II and GHHE-III, and the GHHE-II is a fraction having a lower molecular weight than GHHE-I and a higher molecular weight than GHHE-III. GHHE-III is the fraction having the lowest molecular weight and is characterized by being composed of small-form glucans, poorly differentiated HG, proteins, polyphenols, *etc*.

The enzyme-treated polysaccharide and the purified fractions GHHE-I, GHHE-II, and GHHE-III are obtained from the same polysaccharide, but the types or contents of sugars constituting them may differ. The enzyme-treated polysaccharide of the present disclosure may comprise a neutral sugar, a uronic acid, a protein, a KDO-like material, and a polyphenol, and specifically may be GHHE-0, and more specifically may comprise 68.1% to 73.5% neutral sugar, 11.4% to 14.8% uronic acid, 5.6% to 6.8% protein, 2.1% to 2.9% KDO-like material, and 6.4% to 8.4% polyphenol.

The purified fraction of the present disclosure may comprise a neutral sugar, a uronic acid, a protein, a KDO-like material, and a polyphenol; specifically may be GHHE-I; and more specifically may comprise 66.1% to 80.7% neutral sugar, 16.2% to 19% uronic acid, 1.7% to 1.9% protein, 0.8% to 2.6% KDO-like material, and 4.9% to 6.1% polyphenol.

The term "neutral sugar" refers to sugars having neutral properties, and may be comprised in an amount of 65% to 85% or 66% to 82%, specifically 66.1% to 80.7%, 70% to 74%, 70% to 73.5%, 71% to 75%, 72% to 75%, or 73% to 75%, more specifically 73% to 73.5%, and still more specifically 73.4%.

The term "uronic acid" is also referred to as a sugar acid, and means a monosaccharide in which one or both ends are substituted with a carboxyl group. In the present disclosure, the uronic acid may be comprised in an amount of 10% to 20% or 11% to 19%, specifically 16.2% to 19%, 16% to 18%, or 17% to 18%, more specifically 17.5% to 18%, and still more specifically 17.6%.

The protein may be comprised in an amount of 1% to 7% or 1.5% to 6.8%, specifically 1.5% to 3%, more specifically 1.5% to 2.5% or 1.5% to 2%, and still more specifically 1.8%.

The term "ketodeoxy-octonate (KDO)-like material" refers to all substances corresponding to ketodeoxy-octonate, and in one example, may comprise 2-keto-3-deoxy-D-manno-octulosonic acid. In particular, KDO is an eight-carbon sugar and is one of the components constituting lipopolysaccharide (LPS) of Gram-negative bacteria. By measuring KDO, it is possible to confirm the extent to which immunity can be activated. Specifically, KDO may be comprised in an amount of 0.5% to 3% or 0.5% to 2.5%, specifically 0.8% to 2.6%, 1% to 2.5%, or 1.5% to 2.5%, more specifically 1.5% to 2%, and still more specifically 1.7%.

The term "polyphenol" refers to a type of aromatic alcohol compound found in plants that has at least two phenol groups in a single molecule, and is characterized by having multiple hydroxyl groups as functional groups. The polyphenol may be comprised in the composition of the present disclosure in an amount of 4% to 9%, 4.5% to 8.5%, or 4.6% to 6.3%, specifically 4.9% to 6.1%, 4.6% to 6%, 5% to 6%, or 5.3% to 5.8%, and more specifically 5.5%.

The neutral sugar, uronic acid, protein, KDO-like material, and polyphenol comprised in the purified fraction may be analyzed by methods such as the phenol-H₂SO₄ assay, uronic acid assay, KDO assay, Bradford assay, and polyphenol assay, and any known analytical method may be used without limitation.

The enzyme-treated polysaccharide or purified fraction thereof of the present disclosure may comprise at least one selected from the group comprising rhamnose, fucose, arabinose, mannose, galactose, glucose, glucuronic acid, and galacturonic acid.

The enzyme-treated polysaccharide may comprise 2.7 mol% to 3.7 mol% rhamnose, 8.1 mol% to 8.7 mol% arabinose, 2.3 mol% to 2.7 mol% mannose, 35.8 mol% to 36.8 mol% galactose, 39.5 mol% to 39.9 mol% glucose, and 9.5 mol% to 10.5 mol% galacturonic acid.

The purified fraction may comprise 3.5 mol% to 4.5 mol% rhamnose, 13.9 mol% to 14.5 mol% arabinose, 1.5 mol% to 1.7 mol% mannose, 69.9 mol% to 71.7 mol% galactose, 1.1 mol% to 2.1 mol% glucose, 1.4 mol% to 1.6 mol% glucuronic acid, and 5 mol% to 7.4 mol% galacturonic acid.

The component sugars comprised in the enzyme-treated polysaccharide or purified fraction may be analyzed by, for example, the PMP component sugar method, and any known analytical method may be used without limitation.

The enzyme-treated polysaccharide or purified fraction thereof may be characterized by activating macrophages, splenocytes, or natural killer cells.

The term "macrophage" refers to a cell that regulates immune phenomena through the secretion of various cytokines during the processes of phagocytosing and eliminating bacteria or foreign substances and plays a central role in immune responses to antigens. Macrophages are associated with antigen presentation and non-specific immune actions of lymphocytes, and also exhibit direct cytotoxic activity against tumor cells. In addition, they are known to produce cytokines such as IL-1, IL-6, IL-10, IL-12, and TNF-α that can regulate secondary immune responses. Among these, IL-12 is known as a cytokine that activates NK cells and induces Th1-type immune responses, thereby enhancing responsiveness to foreign cellular substances.

The term "natural killer (NK) cell" refers to a cell that plays an important role in an innate immune response by directly attacking cells infected by a host or tumor cells, and is activated by IFNs or cytokines derived from macrophages. Activated NK cells secrete cytokines such as IFN-γ and TNF-α, thereby promoting apoptosis of tumor cells or contributing to the activation of other immune cells. In particular, regulation of NK cell activity is known to be associated with various diseases such as cancer or immune disorders.

The enzyme-treated polysaccharide or purified fraction thereof of the present disclosure may increase secretion of immune-related cytokines, specifically increase the secreting-ability of at least one selected from the group consisting of IL-2, IFN-γ, IL-4, IL-10, TNF-α, IL-12, and IL-6, and more specifically increase the secreting-ability of the cytokine IL-10 relative to that of a polysaccharide.

In one example of the present disclosure, an enzyme-treated polysaccharide comprising 2.7 mol% to 3.7 mol% rhamnose, 8.1 mol% to 8.7 mol% arabinose, 2.3 mol% to 2.7 mol% mannose, 35.8 mol% to 36.8 mol% galactose, 39.5 mol% to 39.9 mol% glucose, and 9.5 mol% to 10.5 mol% galacturonic acid was confirmed to have a superior immunoenhancing activity compared to polysaccharides comprising different types or contents of component sugars.

In particular, the purified fraction GHHE-I comprising 3.5 mol% to 4.5 mol% rhamnose, 13.9 mol% to 14.5 mol% arabinose, 1.5 mol% to 1.7 mol% mannose, 69.9 mol% to 71.7 mol% galactose, 1.1 mol% to 2.1 mol% glucose, 1.4 mol% to 1.6 mol% glucuronic acid, and 5 mol% to 7.4 mol% galacturonic acid was confirmed to have a significantly superior IL-10 secreting-ability compared to the polysaccharides, enzyme-treated polysaccharides, and purified fractions GHHE-II and GHHE-III comprising different types or contents of component sugars, and thus a remarkably superior immunoenhancing activity.

The composition of the present disclosure may further comprise a mixed extract of angelica, cnidium, and paeonia in an enzyme-treated polysaccharide derived from a mixed extract of angelica, cnidium, and paeonia; or a purified fraction thereof.

As used herein, the term "mix" refers to mixing of the extracts of angelica, cnidium, and paeonia prepared in the present disclosure with an enzyme-treated polysaccharide or purified fraction thereof obtained by treating each of the extracts with a predetermined solvent and treating with an enzyme or further purifying the same according to molecular weight, and the mixing ratio thereof is not particularly limited.

The composition uses extracts of each plant and an enzyme-treated polysaccharide thereof or purified fraction thereof in combination, and can thereby achieve remarkably superior effects compared to a case comprising only an extract of one plant or a polysaccharide thereof, or a case comprising a polysaccharide in an extract. In addition, the composition can exhibit a remarkably superior immunity augmenting or strengthening effect compared to a case comprising only extracts of plants other than angelica, cnidium, and paeonia, or polysaccharides thereof; or a case comprising only a polysaccharide in an extract.

When the mixture mixes an extract with an enzyme-treated polysaccharide or purified fraction thereof, the extract and the enzyme-treated polysaccharide or purified fraction thereof may be comprised at a weight ratio of about 40 to 80:20 to 60, specifically a weight ratio of 45 to 75:25 to 55, more specifically a weight ratio of 50 to 70:30 to 50, still more specifically a weight ratio of 60 to 70:30 to 40, and still more specifically a weight ratio of 60:40, but the weight ratio is not limited thereto.

The enzyme-treated polysaccharide derived from an extract of angelica, cnidium, and paeonia or a purified fraction thereof, or a composition comprising the same may be comprised in an amount of 0.0001 wt% to 99 wt% based on the total weight of the composition, specifically 0.0001 wt% to 90 wt%, more specifically 30 wt% to 80 wt%, still more specifically 35 wt% to 70 wt%, and still more specifically 40 wt% to 65 wt% based on the total weight of the composition, but is not limited thereto.

As used herein, the term "improvement" means enhancing, augmenting, or strengthening immunity by applying the enzyme-treated polysaccharide derived from an extract of angelica, cnidium, and paeonia or a purified fraction thereof of the present disclosure to a composition.

As used herein, the term "anti-cancer immunomodulation" refers to anti-cancer immunomodulation through NK cell activation against tumor cells.

The term "cancer" refers to a disease caused by hyperproliferation of cells when the balance of normal cell apoptosis is broken. In some cases, such abnormal hyperproliferative cells invade surrounding tissues and organs to form a mass and destroy or deform the normal structure of the body, and such a condition is called cancer.

The types of cancer include cerebrospinal tumor, brain tumor, head and neck cancer, lung cancer, breast cancer, thymoma, mesothelioma, esophageal cancer, colorectal cancer, liver cancer, gastric cancer, pancreatic cancer, bile duct cancer, renal cancer, bladder cancer, prostate cancer, testicular cancer, germ cell tumor, ovarian cancer, cervical cancer, endometrial cancer, lymphoma, acute leukemia, chronic leukemia, multiple myeloma, sarcoma, malignant melanoma, and skin cancer, but are not limited thereto.

NK cells are capable of distinguishing cancer cells from normal cells through various immune receptors present on their surface, and therefore exhibit selective cytotoxicity toward cancer cells. Through numerous studies, NK cells have been elucidated to play a key role in eliminating cancer cells, and it has been reported that cancer patients exhibit defects in the number or activity of NK cells (Hanyang Med Rev 2013: 59-64). Accordingly, an increase in NK cell activity may lead to the prevention, amelioration, or treatment of cancer through the elimination of cancer cells.

In a specific embodiment of the present disclosure, it was confirmed that the enzyme-treated polysaccharide or purified fraction thereof of the present disclosure exhibits an NK cell-activating effect against tumor cells, which suggests that a composition comprising, as an active ingredient, the enzyme-treated polysaccharide or purified fraction thereof may be useful for modulating anti-cancer immunity.

The composition may add an enzyme-treated polysaccharide derived from an extract of angelica, cnidium, and paeonia or a purified fraction thereof to a food composition for the purpose of enhancing, improving, augmenting, or strengthening immunity, or modulating anti-cancer immunity; and the food composition may be a health functional food composition.

The food composition may contain a sitologically acceptable carrier.

The food composition of the present disclosure includes all forms such as functional food, nutritional supplement, health food, and food additive, and the above types of food composition may be prepared in various forms according to conventional methods known in the art.

In a case where an enzyme-treated polysaccharide of an extract of angelica, cnidium, and paeonia or a purified fraction thereof is used as a food additive, the enzyme-treated polysaccharide of an extract of angelica, cnidium, and paeonia or a purified fraction thereof may be added as is or may be used together with other foods or food ingredients, and may be used appropriately according to conventional methods. The amount of the active ingredient mixed may be suitably determined depending on the purpose of use (prevention, health maintenance, or therapeutic treatment). Generally, when a food or beverage is prepared, the polysaccharide of an extract of angelica, cnidium, and paeonia is added to the raw material composition in an amount of 0.0001 wt% to 90 wt%, preferably 0.001 wt% to 50 wt%. However, in the case of long-term intake for health and hygiene purposes or health control purposes, the amount may be below the above range.

The type of food is not particularly limited. Examples of the food to which the substances can be added include meat, sausage, bread, chocolate, candy, snacks, confectionery, pizza, ramen, other noodles, gum, dairy products including ice cream, various soups, beverages, tea, drinks, alcoholic beverages, and vitamin complexes, and may include all health foods in the conventional sense.

The health beverage composition of the present disclosure may contain various flavoring agents, natural carbohydrates, *etc*. as additional ingredients, like conventional beverages. The natural carbohydrates described above are monosaccharides such as glucose and fructose, disaccharides such as maltose and sucrose, polysaccharides such as dextrin and cyclodextrin, and sugar alcohols such as xylitol, sorbitol, and erythritol. As sweeteners, natural sweeteners such as thaumatin and stevia extract or synthetic sweeteners such as saccharin and aspartame may be used. The proportion of the natural carbohydrates may be generally about 0.001 to 50 parts by weight, specifically about 0.01 to 30 parts by weight, per 100 parts by weight of the composition of the present disclosure.

In addition to the above, the composition of the present disclosure may contain various nutrients, vitamins, electrolytes, flavoring agents, coloring agents, pectic acid and its salts, alginic acid and its salts, organic acids, protective colloid thickeners, pH adjusters, stabilizers, preservatives, glycerin, alcohols, carbonating agents used in carbonated beverages, *etc*. The proportion of these additives is not greatly important, but is generally selected in the range of 0.01 to 30 parts by weight per 100 parts by weight of the composition of the present disclosure. In addition, the composition of the present disclosure may contain fruit pulp for the preparation of natural fruit juice, fruit juice beverages, and vegetable beverages. The proportion of the pulp is not greatly important, but is generally selected in the range of 0.01 to 30 parts by weight per 100 parts by weight of the composition of the present disclosure, and these ingredients may be used independently or in combination.

Another aspect of the present disclosure to achieve the above objects provides a health supplement comprising a composition for enhancing immunity or modulating anti-cancer immunity, comprising, as an active ingredient, an enzyme-treated polysaccharide of a mixed extract of angelica, cnidium, and paeonia; or a purified fraction thereof.

The terms "angelica", "cnidium", "paeonia", "mixed extract", "polysaccharide", "enzyme-treated product", "purified fraction", "enhancing immunity", and "anti-cancer immunomodulation" are as described above.

The term "health supplement" may include all substances known in the art that serve a supplementary role for health.

Still another aspect of the present disclosure to achieve the above objects provides a method for activating cells *in vitro*, *ex vivo*, or both, comprising treating cells with an enzyme-treated polysaccharide of a mixed extract of angelica, cnidium, and paeonia; or a purified fraction thereof.

The terms "angelica", "cnidium", "paeonia", "mixed extract", "polysaccharide", "enzyme-treated product", "purified fraction", "enhancing immunity", and "anti-cancer immunomodulation" are as described above.

The term "cells" may be macrophages, splenocytes, NK cells, or a combination thereof.

In the present disclosure, the treatment with an enzyme-treated polysaccharide or purified fraction thereof may be performed according to a conventional method in the art, and the treatment concentration of an enzyme-treated polysaccharide or purified fraction thereof may be 1 µM to 100 µM, 1 µM to 90 µM, 1 µM to 80 µM, 1 µM to 70 µM, or 1 µM to 60 µM, but is not limited thereto.

In the present disclosure, "*in vitro*" refers to an experimental process performed in a controllable environment, such as a test tube, rather than within a living organism. In the *in vitro* environment, various variables can be easily controlled, thereby enabling efficient confirmation of results.

In the present disclosure, "*ex vivo*" refers to a process of experimenting on cells or tissues isolated from an organism in an extracorporeal environment. Through *ex vivo* experiments, it is possible to maintain responses similar to those in a living organism while enabling precise manipulation.

In a specific embodiment of the present disclosure, it was confirmed that treating macrophages, splenocytes, and NK cells with the enzyme-treated polysaccharide or purified fraction thereof increases secretion levels of various cytokines associated with the cells, which suggests that the enzyme-treated polysaccharide or purified fraction thereof of the present disclosure can activate macrophages, splenocytes, and NK cells *in vitro*.

Still another aspect of the present disclosure to achieve the above objects provides a use of a composition comprising, as an active ingredient, an enzyme-treated polysaccharide of a mixed extract of angelica, cnidium, and paeonia; or a purified fraction thereof for enhancing immunity or modulating anti-cancer immunity.

Still another aspect of the present disclosure to achieve the above objects provides a method for enhancing immunity or modulating anti-cancer immunity, comprising administering to a subject a composition comprising, as an active ingredient, an enzyme-treated polysaccharide of a mixed extract of angelica, cnidium, and paeonia; or a purified fraction thereof.

The terms "mixed extract", "enzyme-treated polysaccharide", and "purified fraction" are as described above.

As used herein, the term "administration" refers to introducing the composition of the present disclosure into a subject by a suitable method, and the administration may be performed using various oral or parenteral routes, as long as it enables the composition to reach a target tissue.

In the present disclosure, the subject may include a human or a non-human species, more specifically mammals including humans, and even more specifically humans, dogs, rats, rabbits, cats, horses, cows, *etc*. However, any subject may be included without limitation as long as fatigue alleviation or enhancement of exercise performance can be achieved by administration of the composition of the present disclosure.

Hereinafter, the present disclosure will be described in more detail with reference to Examples. However, these Examples are intended to exemplify the present disclosure, and the scope of the present disclosure is not limited by these Examples, as will be apparent to those skilled in the art to which the present disclosure pertains.

### Example 1: Preparation of mixed extract of angelica, cnidium, and paeonia; polysaccharide thereof; enzyme-treated polysaccharide; and purified fraction thereof

### Example 1-1: Preparation of mixed extract of Angelica sinensis (Oliv.) Diels, Ligusticum chuanxiong Hort., and Paeonia lactiflora Pallas

The root of *Angelica sinensis* (Oliv.) Diels, the rhizome of *Ligusticum chuanxiong* Hort., and the root of *Paeonia lactiflora* Pallas were each dried in the shade, cut into pieces, and mixed. Distilled water amounting to 10 times the total weight of each herbal medicinal ingredient was added, and hot water extraction was performed at 95°C for 4 hours to obtain an extract. In particular, *Angelica sinensis* (Oliv.) Diels, *Ligusticum chuanxiong* Hort., and *Paeonia lactiflora* Pallas were mixed at a weight ratio of 1:1.6:1.3, respectively. The hot water extract was concentrated under reduced pressure, and filtered to obtain a mixed extract of *Angelica sinensis* (Oliv.) Diels, *Ligusticum chuanxiong* Hort., and *Paeonia lactiflora* Pallas.

### Example 1-2: Preparation of ethanol polysaccharide of mixed extract

An ethanol polysaccharide (GHHW-P) was prepared from the mixed extract prepared in Example 1-1. The ethanol polysaccharide GHHW-P is a crude polysaccharide obtained by precipitation with ethanol without further purification.

Specifically, to the mixed hot water extract prepared in Example 1-1, 95% ethanol was added and the mixture was left to stand at 25°C or less for 16 hours, and then centrifugation was performed to obtain a precipitated ethanol polysaccharide.

### Example 1-3: Preparation of enzyme-treated polysaccharide and three purified fractions thereof from ethanol polysaccharide

### Example 1-3-1: Confirmation of purity of ethanol polysaccharide

The purity of the ethanol polysaccharide (GHHW-P) obtained in Example 1-2 was determined to examine whether any mixed substances were present in the ethanol polysaccharide.

Specifically, size-exclusion chromatography was performed using a refractive index detector-HPLC (RID-HPLC) equipped with a Superdex 75 GL column pre-packed with a dextran-based porous resin.

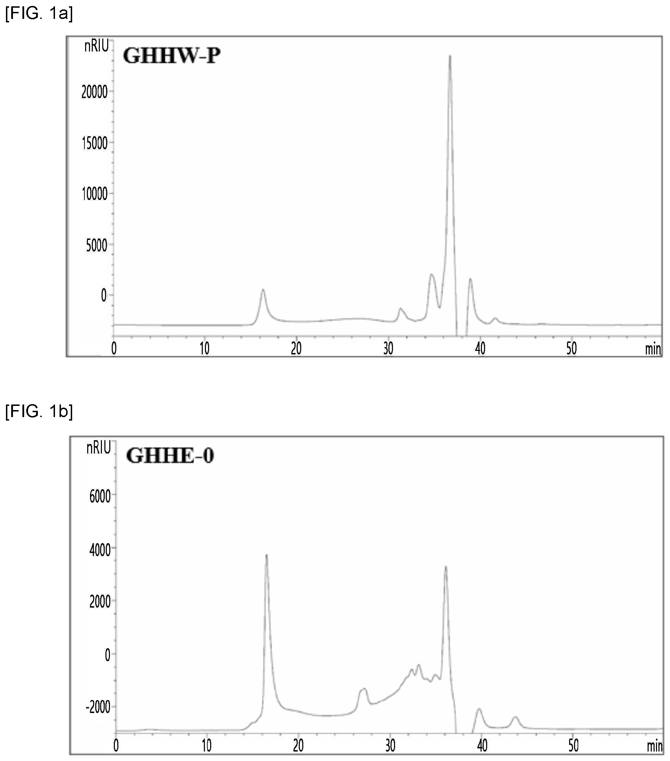

As a result, as shown in FIG. 1a, a peak corresponding to a relatively high molecular weight was detected first at around 14 minutes, followed by detection of a peak corresponding to a relatively low molecular weight after 30 minutes.

These results indicate that the ethanol polysaccharide GHHW-P of a mixed extract of *Angelica sinensis* (Oliv.) Diels, *Ligusticum chuanxiong* Hort., and *Paeonia lactiflora* Pallas is in a crude state in which various substances are mixed, and it is therefore necessary to identify the active moiety that actually exhibits activity in the ethanol polysaccharide of the mixed extract. To this end, an enzyme-treated product obtained by treating the obtained ethanol polysaccharide with an enzyme and purified fractions obtained by purifying the same were respectively prepared.

### Example 1-3-2: Preparation of enzyme-treated polysaccharide

The ethanol polysaccharide GHHW-P of a mixed extract obtained in Example 1-3-1 was de-esterified using NaOH, and then the pH was adjusted to 6 using HCl, which is the optimal reaction pH for polygalacturonase, a pectinase. Thereafter, polygalacturonase was added and allowed to react at 50°C for 72 hours, after which the enzyme reaction was terminated by heating, followed by centrifugation to separate the precipitated enzyme from the reaction solution. The reaction solution was then dialyzed for 3 days using a dialysis tubing bag (MW cut-off: 14,000 Da) to remove low molecular weight fragments generated from the enzyme reaction, and subsequently concentrated and freeze-dried to obtain an enzyme-treated polysaccharide, GHHE-0. Thereafter, the enzyme was replaced with cellulase, α-amylase, and protease to obtain enzyme-treated polysaccharides GHHE-C (GHHW-P + cellulase), GHHE-α (GHHW-P + α-amylase), and GHHE-pro (GHHW-P + protease), respectively, using the same method.

To verify whether the enzyme treatment was properly performed, the elution pattern of the enzyme-treated polysaccharide was analyzed by size-exclusion chromatography using HPLC. As a result, as shown in FIG. 1b, an increase in peaks in the 25 to 30-minute range was observed, indicating that the low molecular weight fraction relatively increased compared to the highest molecular weight fraction due to the enzyme treatment. Such results indicate that the enzyme treatment of the polysaccharide was successfully performed.

### Example 1-3-3: Preparation of three purified fractions

Using the enzyme-treated polysaccharide GHHE-0 obtained in Example 1-3-2, purified fractions were obtained through a purification process. Specifically, swollen Sephadex G-75 resin was packed into an open column (diameter: 2.5 cm, length: 90 cm) and equilibrated with 50 mM ammonium formate buffer (pH 5.5). The previously obtained enzyme-treated ethanol polysaccharide GHHE-0 was then loaded onto the column and separated into 100 fractions. The chemical characteristics of these fractions, including neutral sugars, uronic acids, proteins, and KDO, were analyzed, thereby isolating three purified fractions having different molecular weights.

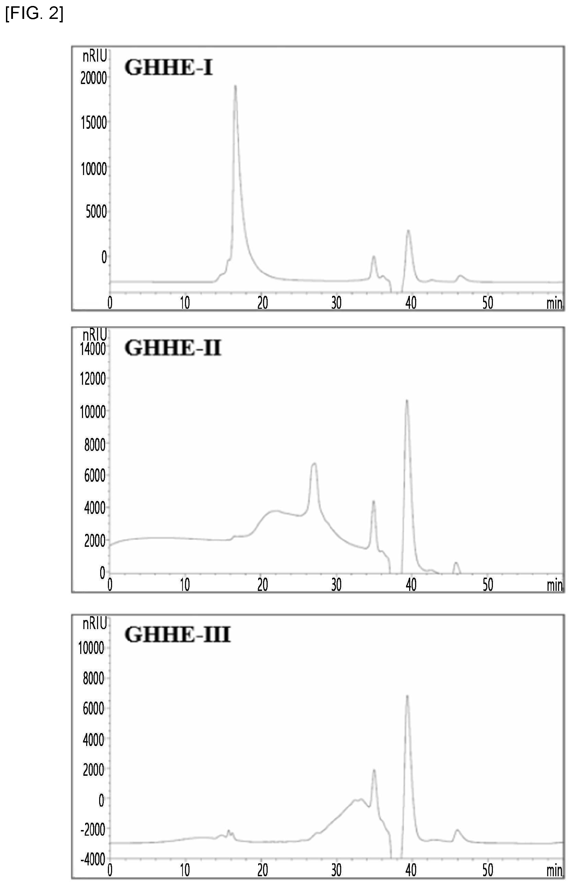

The three purified fractions were named GHHE-I, GHHE-II, and GHHE-III, respectively, and are illustrated in FIG. 2.

As a result, as shown in FIG. 2, taking into account that substances having higher molecular weights appear earlier and substances having lower molecular weights appear later in GPC analysis, it can be seen from the peak patterns of the three purified fractions that from the high molecular weight fraction GHHE-I to the low molecular weight fraction GHHE-III, the main peak progressively shifts toward the later, lower molecular weight region. Through this, it can be confirmed that GHHE-I, GHHE-II, and GHHE-III were well-fractionated according to molecular weight. In addition, in the case of GHHE-I, it was confirmed that the main peak exhibits a sharp, symmetric single peak. These results indicate that, among the three purified fractions, GHHE-I has the highest degree of purification and exhibits the highest molecular weight peak compared to GHHE-II and GHHE-III.

Thereafter, in order to obtain sufficient quantities to confirm various characteristics and immunoenhancing activities of the three purified fractions, the above procedure was repeated at least ten times.

### Example 2: Analysis of chemical composition and component sugar composition in five materials, i.e., polysaccharide, enzyme-treated polysaccharide, and purified fractions

The chemical compositions and component sugar compositions of a total of five materials, *i.e*., the polysaccharide, enzyme-treated polysaccharide, and purified fractions thereof isolated or purified in Example 1 (GHHW-P, GHHE-0, GHHE-I, GHHE-II, and GHHE-III) were each analyzed and illustrated in Tables 1 and 2.

### Example 2-1: Analysis of chemical composition

The chemical composition was determined by analyzing neutral sugars, uronic acids, KDO-like materials, proteins, and polyphenols, and the specific methods for analyzing the chemical composition are as follows.

Specifically, for analysis of general components of polysaccharides, the content of neutral sugars was measured by the phenol-sulfuric acid method using galactose as the standard substance (see Dubois, M., Gilles, KA., Hamilton, JK., Rebers, PA., Smith FAJN. (1951) A colorimetric method for the determination of sugars. Nature, 168(4265): 167.), and the content of uronic acids was measured by the m-hydroxydiphenyl method using galacturonic acid as the standard substance (see Blumenkrantz N, Gustav AH. New method for quantitative determination of uronic acids. Analytical Biochemistry 54 (1973): 484-489.).

The content of 2-keto-3-deoxy-D-manno-octulosonic acid (KDO) was analyzed by the thiobarbituric acid (TBA) method. Specifically, since KDO has very weak bonds, sugars were hydrolyzed by heating with 0.4 N dilute sulfuric acid, followed by addition of 0.04 M HIO₄ to reduce hydroxyl (-OH) groups and induce ring opening, thereby forming malondialdehyde groups. Then, 2% Na₂SO₃/0.5 M HCl was added for decolorization, and 2-thiobarbituric acid (TBA) was added to react with malondialdehyde to form a redcolored chromogen. Subsequently, DMSO was added to stabilize the colorimetric reaction of the chromogen, thereby allowing identification of KDO-like materials.

Proteins were analyzed by the Bradford method using bovine serum albumin (BSA) as the standard substance. Specifically, the analysis was performed by allowing Coomassie dye ligand to bind to positively charged regions of proteins (*i.e*., arginine, lysine, and histidine) to produce a blue color.

Finally, the content of phenolic compounds was quantified and analyzed by the Folin-Ciocalteu method using gallic acid as the standard substance. Specifically, each of the standards and samples was prepared, and standards were pre-prepared in Eppendorf tubes at concentrations of 0, 200, 400, 600, 800, and 1000 µg/mL. Eppendorf tubes for standards and samples were prepared in triplicate, and 790 µL of ionized water (IW) was added to each tube, followed by addition of 10 µL of the corresponding standard or sample. Then, 500 µL of Folin-Ciocalteu reagent was added to each tube and allowed to stand for 1 minute, after which 150 µL of 20% sodium carbonate was added and vortexed. Immediately after vortexing, the tube caps were opened and the tubes were left standing in the dark for 2 hours. Subsequently, 200 µL aliquots were collected and measured at a wavelength of 750 nm to determine the polyphenol content.

The results are as shown in Table 1 below.

**[Table 1]**

| Chemical Properties (%) | GHHW-P | GHHE-0 | GHHE-I | GHHE-II | GHHE-III |
|---|---|---|---|---|---|
| Neutral sugar | 76.5±0.2 | 70.8±2.7 | 73.4±7.3 | 63.9±1.5 | 51.4±4.6 |
| Uronic acid | 12.5±2.3 | 13.1±1.7 | 17.6±1.4 | 19.0±1.2 | 12.6±1.5 |
| Protein | 1.2±0.2 | 6.2±0.6 | 1.8±0.1 | 5.5±0.3 | 10.4±0.2 |
| KDO-like materials | 5.9±7.2 | 2.5±0.4 | 1.7±0.9 | 2.1±1.9 | 5.5±0.7 |
| Polyphenol | 4.0±0.5 | 7.4±1.0 | 5.5±0.6 | 9.5±1.5 | 20.1±2.6 |

As a result, as shown in Table 1, the ethanol polysaccharide GHHW-P was composed of 76.5 ± 0.2% neutral sugars and 12.5 ± 2.3% uronic acids, confirming that it consists mostly of sugars.

For comparison, the chemical compositions of one enzyme-treated polysaccharide and three purified fractions were analyzed. As shown in Table 1, the chemical composition of GHHE-0 was found to be 70.8 ± 2.7% neutral sugars, 13.1 ± 1.7% uronic acids, 6.2 ± 0.6% proteins, 2.5 ± 0.4% KDO-like materials, and 7.4 ± 1.0% polyphenols.

Further, in the purified fractions, neutral sugar contents were 73.4 ± 7.3% in GHHE-I, 63.9 ± 1.5% in GHHE-II, and 51.4 ± 4.6% in GHHE-III, which were reduced relative to the polysaccharide. Uronic acid contents were 17.6 ± 1.4% in GHHE-I, 19.0 ± 1.2% in GHHE-II, and 12.6 ± 1.5% in GHHE-III, which were increased relative to the polysaccharide, but the increase was more pronounced in GHHE-I and GHHE-II than in GHHE-III. Notably, in GHHE-I, the contents of proteins and polyphenols were not markedly increased and remained at levels similar to those of the polysaccharide, whereas in GHHE-II and GHHE-III, the contents of proteins and polyphenols were significantly increased relative to those in the ethanol polysaccharide. This confirmed that, although GHHE-I, GHHE-II, and GHHE-III are purified fractions obtained from purifying the ethanol polysaccharide, their chemical compositions are completely different from that of the polysaccharide.

### Example 2-2: Analysis of component sugar composition

Next, the component sugar composition was also analyzed by an HPLC analysis method using 1-phenyl-3-methyl-5-pyrazolone (PMP) labeling, and the component sugars were identified as rhamnose, fructose, arabinose, xylose, mannose, galactose, glucose, glucuronic acid, and galacturonic acid. The specific method for analysis is as follows.

Specifically, each sample was dissolved at a concentration of 1 mg/mL, and 500 µL thereof was transferred to a cap tube, dried by N₂ flushing, and then 1 mL of 2 M trifluoroacetic acid (TFA) was added. The tube was wrapped with Teflon tape and reacted at 121°C for 90 minutes. After cooling, the sample was dried by N₂ flushing, followed by addition of 200 µL of methanol and drying by N₂ flushing. Based on 10 samples, 100 µL of 0.3 M NaOH and 120 µL of 0.5 M PMP (in MeOH) were added (PMP, MW 174.2: 130 mg/1.5 mL) and reacted at 70 °C for 100 minutes. After sufficient cooling, the reaction was neutralized with 100 µL of 0.3 M HCl. Then, after N₂ flushing, 0.5 mL each of water and chloroform were added and shaken. After centrifuging at 2500 rpm for 5 minutes, the upper aqueous layer was collected and transferred to a 2 mL Eppendorf tube, to which chloroform was added and centrifuged three times. The resulting solution was filtered through a 0.45 µM filter. Finally, each component sugar was measured by HPLC, and the results are shown in Table 2 below.

**[Table 2]**

| Chemical Properties (%) | GHHW-P | GHHE-0 | GHHE-I | GHHE-II | GHHE-III |
|---|---|---|---|---|---|
| Component sugar (PMP) | (Mole%) | | | | |
| Rhamnose | - | 3.2±0.0 | 4.0±0.0 | 5.0±0.4 | 4.5±0.5 |
| Fucose | - | - | - | 0.6±0.2 | - |
| Arabinose | 2.9±0.5 | 8.4±0.3 | 14.2±0.3 | 15.6±0.8 | 7.4±0.3 |
| Xylose | - | - | - | - | - |
| Mannose | 0.7±0.0 | 2.5±0.2 | 1.6±0.1 | 2.3±0.3 | 8.8±2.8 |
| Galactose | 7.9±0.1 | 36.3±0.0 | 70.8±0.9 | 56.6±0.3 | 23.7±1.5 |
| Glucose | 76.9±3.3 | 39.7±0.2 | 1.6±0.0 | 8.3±0.2 | 21.4±1.7 |
| Glucuronic acid | - | - | 1.5±0.1 | - | - |
| Galacturonic acid | 11.6±3.7 | 10.0±0.0 | 6.2±1.2 | 11.6±1.0 | 34.3±1.2 |

As a result, as shown in Table 2, the component sugar composition analysis revealed that the ethanol polysaccharide GHHW-P comprised 76.9 ± 3.3% glucose, 11.6 ± 3.7% galacturonic acid, 7.9 ± 0.1% galactose, and 2.9 ± 0.5% arabinose, and comprised mannose in a small amount of 0.7%.

Meanwhile, the component sugar composition of GHHE-0 was found to be 3.2% rhamnose, 8.4 ± 0.3% arabinose, 2.5 ± 0.2% mannose, 36.3% galactose, 39.7 ± 0.2% glucose, and 10.0% galacturonic acid, confirming that its composition is markedly different from that of the polysaccharide GHHW-P.

Further, in the purified fractions, GHHE-I was found to comprise 70.8 ± 0.9% galactose, 14.2 ± 0.3% arabinose, 6.2 ± 1.2% galacturonic acid, and 4.0% rhamnose, to comprise rhamnose at 4.0% which is not present in the polysaccharide, and further to comprise 1.6 ± 0.1% mannose and 1.6% glucose. In addition, it was confirmed that only GHHE-I comprises glucuronic acid at 1.5 ± 0.1%, thereby confirming that the purified fractions also have component sugar compositions completely different from that of the polysaccharide.

In particular, although the types of component sugars comprised in the purified fractions GHHE-II and GHHE-III were not largely different from those of GHHE-I, GHHE-II and GHHE-III contained relatively higher amounts of glucose and galacturonic acid and relatively lower amounts of galactose compared to GHHE-I. Notably, GHHE-II and GHHE-III did not comprise glucuronic acid, and GHHE-II was confirmed to comprise a trace amount of fructose, unlike the other purified fractions. This indicates that, despite obtaining the fractions through purification of the same enzyme-treated polysaccharide, the types and contents of component sugars differ between the fractions. In particular, the GHHE-I fraction was confirmed to comprise multiple high molecular weight component sugars compared to the GHHE-II and GHHE-III fractions.

### Example 3: Confirmation of immunoenhancing and anti-cancer immunomodulatory effects of polysaccharide, enzyme-treated polysaccharide, and purified fractions thereof

To confirm the immunoenhancing effects of the five materials, *i.e*., the ethanol polysaccharide, enzyme-treated polysaccharide, and purified fractions thereof obtained in Example 1, the degrees of activation of macrophages, splenocytes, and NK cells were examined and compared.

### Example 3-1: Confirmation of macrophage activation

Macrophage activation by the five materials, *i.e*., the polysaccharide, enzyme-treated polysaccharide, and purified fractions (GHHW-P, GHHE-0, GHHE-I, GHHE-II, and GHHE-III) was evaluated based on their abilities to secrete cytokines IL-6, IL-12, and TNF-α.

Specifically, peritoneal macrophages were isolated from BALB/c mice, and treated with each of the five materials dissolved at various concentrations. After 24 hours, cytokines secreted into the supernatant were measured using ELISA kits.

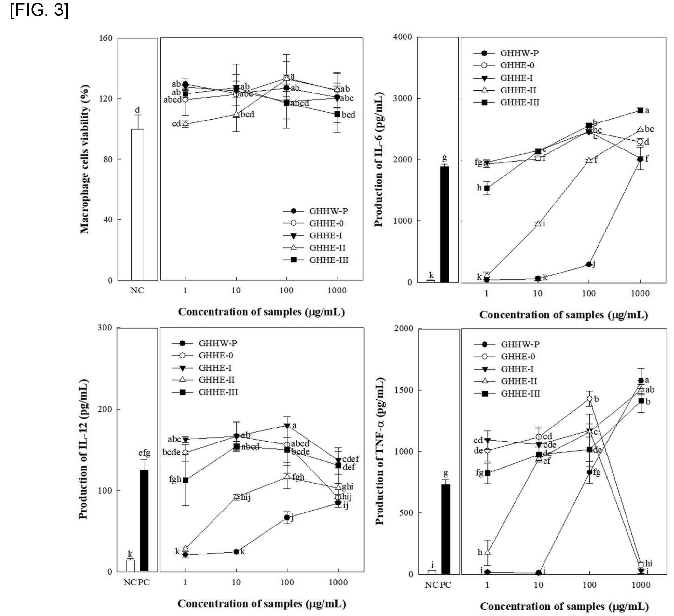

As a result, as shown in FIG. 3, no toxicologically significant differences were observed for any of the five materials, *i.e*., the polysaccharide, enzyme-treated polysaccharide, and purified fractions, within the concentration range of 1 to 1000 µg/mL.

Next, measurement of cytokines secreted into the culture supernatant using ELISA kits confirmed that the ethanol polysaccharide GHHW-P exhibited effects on IL-6, IL-12, and TNF-α secretion only at concentrations of at least 100 µg/mL.

Meanwhile, the enzyme-treated polysaccharide GHHE-0 obtained by treating the ethanol polysaccharide with pectinase, and the three purified fractions GHHE-I, GHHE-II, and GHHE-III obtained by purifying the enzyme-treated polysaccharide, all exhibited secretion effects for the three cytokines at concentrations of at least 10 µg/mL, confirming that macrophage activation occurs even at markedly lower concentrations compared to the ethanol polysaccharide.

In particular, among the purified fractions, GHHE-I exhibited secretion effects for all three cytokines starting at 1 µg/mL in comparison with GHHE-II and GHHE-III, and was confirmed to have the most superior ability to secrete IL-12 and TNF-α at low concentrations of 1 to 10 µg/mL.

From these results, it can be confirmed that, unlike the ethanol polysaccharide in a crude form due to the presence of various mixed substances, the enzyme-treated polysaccharide and the purified fractions obtained therefrom exhibit effective immunoenhancing activity at concentrations that are tenfold lower. Among these, the purified fraction GHHE-I was confirmed to exhibit superior immunoenhancing activity even in comparison with the other purified fractions.

Next, macrophage activation and cytokine producing-abilities were evaluated and compared in cases where different enzymes were used for the enzyme-treated polysaccharide.

Peritoneal macrophages were isolated from BALB/c mice, and treated with each of the ethanol polysaccharide GHHW-P and the four enzyme-treated polysaccharides GHHE-0, GHHE-C, GHHE-α, and GHHE-pro prepared in Example 1-3-2 at concentrations of 1 µg/mL or 10 µg/mL. After 24 hours, cytokines secreted into the supernatant were measured using ELISA kits.

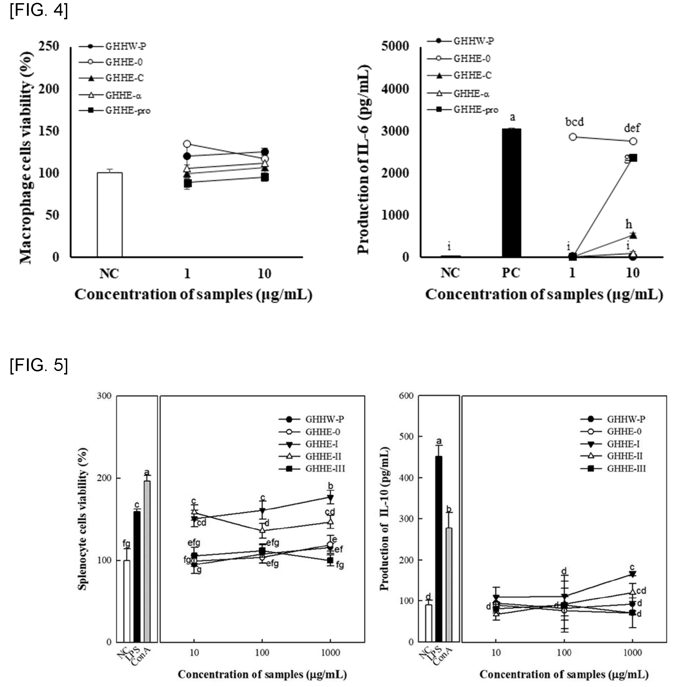

As a result, no toxicologically significant differences were observed between the ethanol polysaccharide and the enzyme-treated polysaccharides, and it was confirmed that GHHE-0 exhibited the highest macrophage viability even at a low concentration of 1 µg/mL. Moreover, measurement of cytokines secreted into each culture supernatant using ELISA kits revealed that GHHE-0 exhibited a markedly superior IL-6 secretion effect compared to the ethanol polysaccharide GHHW-P and the other enzyme-treated products (FIG. 4).

### Example 3-2: Confirmation of splenocyte proliferative ability and cytokine (IL-10) expressing-ability

Next, splenocyte activation by the five materials, *i.e*., the polysaccharide, enzyme-treated polysaccharide, and purified fractions (GHHW-P, GHHE-0, GHHE-I, GHHE-II, and GHHE-III) was evaluated based on splenocyte proliferative ability and the level of IL-10 cytokine expression.

Specifically, splenocytes were isolated from BALB/c mice, and treated with each of the five materials dissolved at various concentrations. After 96 hours, cytokines secreted into the supernatant were measured using ELISA kits.

As a result, as shown in FIG. 5, no toxicologically significant differences were observed for any of the five materials, *i.e*., the polysaccharide, enzyme-treated polysaccharide, or purified fractions, within the concentration range of 10 to 1000 µg/mL. Among them, the purified fractions GHHE-I and GHHE-II were confirmed to significantly promote splenocyte proliferation, and in particular, GHHE-I was confirmed to be superior to GHHE-II.

In terms of cytokine measurement, it was confirmed that GHHE-I exhibits a clear ability to secrete IL-10 compared to the polysaccharide or the purified fractions GHHE-II and GHHE-III. Unlike the polysaccharide, GHHE-II, and GHHE-III, which exhibited an IL-10 secreting-ability of about 100 pg/mL, GHHE-I exhibited a secreting-ability of about 180 pg/mL, indicating an increased IL-10 secreting-ability compared to the polysaccharide or the other purified fractions.

From these results, it can be understood that treatment of a mixed hot water extract of *Angelica sinensis* (Oliv.) Diels, *Ligusticum chuanxiong* Hort., and *Paeonia lactiflora* Pallas with pectinase enzyme enhances immunological activity, and in particular, it was confirmed that the active moiety of the polysaccharide derived from the mixed hot water extract of *Angelica sinensis* (Oliv.) Diels, *Ligusticum chuanxiong* Hort., and *Paeonia lactiflora* Pallas is the purified polysaccharide fraction GHHE-I.

Accordingly, the natural killer cell-activating effect of GHHE-I was evaluated.

### Example 3-3: Confirmation of tumor cell-killing effect of natural killer cells

The effect of the purified fraction GHHE-I, which was identified as the active moiety based on the results of macrophage and splenocyte activation above, on the tumor cell-killing effect of natural killer cells was evaluated.

Specifically, the effect was induced by administering the sample at doses of 0.5, 5, and 50 mg/kg into the tail vein of BALB/c mice. The purified fraction GHHE-I was intravenously injected twice, 3 days and 1 day prior to cell collection. On the day of the experiment, the mice were sacrificed and cells were harvested from the spleen, followed by co-culture with the hematologic malignant cell line YAC-1 lacking MHC class I molecules, to compare and analyze the cytolytic activity of natural killer cells.

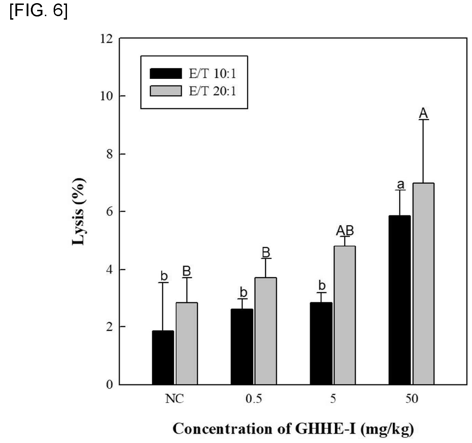

As a result, as shown in FIG. 6, a dose-dependent natural killer cell-activating effect was observed, and in particular, the experimental group administered with 50 mg/kg exhibited at least about a two-fold effect compared to the negative control group administered with only PBS. As used herein, the E/T ratio refers to the ratio of NK cells isolated from the spleen (effector cells, E) to Yac-1 tumor cells (target cells, T) known to be sensitive to NK cells. Since NK cell activity may or may not be clearly exhibited depending on whether an appropriate balance of a specific E/T ratio is achieved, the effect was evaluated at two different E/T ratios.

From these results, it was confirmed that the purified fraction GHHE-I not only induces cytokine secretion representative of immune activation of macrophages and splenocytes, but also increases the tumor cell-cytotoxic effect of natural killer cells known to exhibit tumor cell-specific activity upon *in vivo* administration.

Thus, it was confirmed that, among the purified fractions purified from an enzyme-treated polysaccharide prepared by enzymatic treatment of an ethanol polysaccharide of a mixed hot water extract of *Angelica sinensis* (Oliv.) Diels, *Ligusticum chuanxiong* Hort., and *Paeonia lactiflora* Pallas of the present disclosure, the purified fraction GHHE-I composed of specific types and contents of component sugars is applicable as an enhancer of immunity including innate immunity, and in particular, possesses anti-cancer immunomodulatory ability against tumor cells.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the present disclosure should be interpreted to include all modifications or modified forms derived from the meaning and scope of the claims as set forth below, as well as equivalent concepts, rather than from the detailed description above.

## Claims

1. A composition for enhancing immunity, comprising, as an active ingredient, an enzyme-treated polysaccharide of a mixed extract of angelica, cnidium, and paeonia; or a purified fraction thereof.

2. The composition according to claim 1, wherein the immunity is innate immunity, acquired immunity, or a combination thereof.

3. The composition according to claim 1, wherein the mixed extract is extracted with a solvent selected from the group consisting of water, a linear or branched alcohol having 1 to 6 carbon atoms, an organic solvent, and a mixed solvent thereof.

4. The composition according to claim 1, wherein the mixed extract comprises angelica, cnidium, and paeonia at a weight ratio of 1:1.2 to 3:1.2 to 3.

5. The composition according to claim 1, wherein the mixed extract is a mixed extract of *Angelica sinensis* (Oliv.) Diels, *Ligusticum chuanxiong* Hort., and *Paeonia lactiflora* Pallas.

6. The composition according to claim 1, wherein the polysaccharide is an ethanol polysaccharide.

7. The composition according to claim 1, wherein the enzyme comprises pectinase.

8. The composition according to claim 1, wherein the enzyme-treated polysaccharide or purified fraction thereof comprises a neutral sugar, a uronic acid, a protein, a ketodeoxy-octonate (KDO)-like material, and a polyphenol.

9. The composition according to claim 8, wherein the enzyme-treated polysaccharide comprises 68.1% to 73.5% neutral sugar, 11.4% to 14.8% uronic acid, 5.6% to 6.8% protein, 2.1% to 2.9% KDO-like material, and 6.4% to 8.4% polyphenol.

10. The composition according to claim 8, wherein the purified fraction comprises 66.1% to 80.7% neutral sugar, 16.2% to 19% uronic acid, 1.7% to 1.9% protein, 0.8% to 2.6% KDO-like material, and 4.9% to 6.1% polyphenol.

11. The composition according to claim 1, wherein the enzyme-treated polysaccharide or purified fraction thereof is at least one selected from the group comprising rhamnose, fucose, arabinose, mannose, galactose, glucose, glucuronic acid, and galacturonic acid.

12. The composition according to claim 11, wherein the enzyme-treated polysaccharide comprises 2.7 mol% to 3.7 mol% rhamnose, 8.1 mol% to 8.7 mol% arabinose, 2.3 mol% to 2.7 mol% mannose, 35.8 mol% to 36.8 mol% galactose, 39.5 mol% to 39.9 mol% glucose, and 9.5 mol% to 10.5 mol% galacturonic acid.

13. The composition according to claim 11, wherein the purified fraction comprises 3.5 mol% to 4.5 mol% rhamnose, 13.9 mol% to 14.5 mol% arabinose, 1.5 mol% to 1.7 mol% mannose, 69.9 mol% to 71.7 mol% galactose, 1.1 mol% to 2.1 mol% glucose, 1.4 mol% to 1.6 mol% glucuronic acid, and 5 mol% to 7.4 mol% galacturonic acid.

14. The composition according to claim 1, wherein the enzyme-treated polysaccharide or purified fraction thereof is **characterized by** activating macrophages, splenocytes, or natural killer cells.

15. The composition according to claim 1, wherein the enzyme-treated polysaccharide or purified fraction thereof is **characterized by** increasing immune-related cytokines.

16. The composition according to claim 15, wherein the cytokine is at least one selected from the group consisting of IL-2, IFN-γ, IL-4, IL-10, TNF-α, IL-12, and IL-6.

17. The composition according to claim 1, wherein the composition further comprises a mixed extract of angelica, cnidium, and paeonia.

18. The composition according to claim 17, wherein the mixed extract and the enzyme-treated polysaccharide or purified fraction thereof are mixed at a weight ratio of 60 to 70:30 to 40.

19. The composition according to claim 1, wherein the enzyme-treated polysaccharide or purified fraction thereof is comprised in an amount of 0.001% to 99% by weight based on the total weight of the composition.

20. The composition according to claim 1, wherein the composition is a food composition.

21. The composition according to claim 20, wherein the food composition is prepared as a powder, granule, tablet, capsule, syrup, or beverage.

22. A health supplement comprising the composition according to any one of claims 1 to 21.

23. A method for activating cells *in vitro*, *ex vivo*, or both, comprising treating cells with an enzyme-treated polysaccharide of a mixed extract of angelica, cnidium, and paeonia; or a purified fraction thereof.

24. The method according to claim 23, wherein the cells are macrophages, splenocytes, natural killer cells, or a combination thereof.

25. A use of a composition comprising, as an active ingredient, an enzyme-treated polysaccharide of a mixed extract of angelica, cnidium, and paeonia; or a purified fraction thereof for enhancing immunity.

26. A method for enhancing immunity, comprising administering to a subject a composition comprising, as an active ingredient, an enzyme-treated polysaccharide of a mixed extract of angelica, cnidium, and paeonia; or a purified fraction thereof.
